(19) Europäisches Patentamt
European Patent Office
Office européen des brevets

(11) **EP 2 902 101 A1**

(12) **EUROPEAN PATENT APPLICATION**
published in accordance with Art. 153(4) EPC

(43) Date of publication:
05.08.2015 Bulletin 2015/32

(51) Int Cl.:
*B01J 13/00* (2006.01)       *A23D 7/00* (2006.01)
*A23L 1/30* (2006.01)       *A61K 8/06* (2006.01)
*A61K 8/31* (2006.01)       *A61K 8/67* (2006.01)

(21) Application number: 13840203.7

(22) Date of filing: 27.09.2013

(86) International application number:
PCT/JP2013/076419

(87) International publication number:
WO 2014/051116 (03.04.2014 Gazette 2014/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 28.09.2012 JP 2012217835

(71) Applicant: FUJIFILM Corporation
Tokyo 106-8620 (JP)

(72) Inventors:
• KITAOKA, Hiroyuki
Ashigarakami-gun
Kanagawa 258-8577 (JP)

• KUSUDA, Fumi
Ashigarakami-gun
Kanagawa 258-8577 (JP)
• MORI, Mikinaga
Ashigarakami-gun
Kanagawa 258-8577 (JP)

(74) Representative: Mayall, John
Fujifilm Imaging Colorants Limited
Intellectual Property Group
P.O. Box 42
Hexagon Tower
Blackley, Manchester
M9 8ZS (GB)

(54) **LYCOPENE-CONTAINING OIL-IN-WATER EMULSION COMPOSITION AND PRODUCTION METHOD THEREFOR**

(57) A lycopene-containing oil-in-water emulsion composition and a method of producing the emulsion composition, the emulsion composition includes an oil phase containing lycopene in an amount of from 0.00001 % to 0.1 % by mass or less with respect to a total mass of the emulsion composition, and an aqueous phase containing at least one kind selected from the group consisting of ascorbic acid, derivatives thereof, salts thereof ascorbic acid and derivatives of ascorbic acid, wherein an average particle diameter of emulsion particles is 120 nm or less.

**Description**

Technical Field

The present invention relates to a lycopene-containing oil-in-water emulsion composition and a method for producing the emulsion composition.

Background Art

In recent years, attention has been paid to the high functionality of lycopene, and various compositions containing have been suggested. For example, Japanese Patent Application Laid-Open (JP-A) No. 2011-241177 describes a method for obtaining a carotinoid-containing composition by heating a carotinoid component containing a crystalline carotenoid such as lycopene and an oil phase component mixed liquid containing a specific (poly)glycerin fatty acid ester under a specific temperature condition.

Furthermore, as a technology for improving storage stability by applying ascorbic acid or a derivative, or the like, for example, Patent Literature 2 suggests a technology for incorporating tocopherol or a derivative thereof and ascorbic acid or a derivative thereof and/or a carotinoid into a milk beverage. According to the technology described in JP-A No. 2002-78447, it is considered that the deterioration of a milk beverage filled in a light-transmissive storage container under light irradiation is suppressed.

SUMMARY OF INVENTION

Technical Problem

It is known that lycopene has an excellent antioxidative effect but is oxidized very easily, and thus lycopene may easily decomposeowing to temperature conditions, storage period and the like. Furthermore, since lycopene is an oil-soluble substance, it is sometimes used as an oil-in-water emulsion composition in applications that are applied to water-based compositions. For this kind of oil-in-water emulsion composition containing lycopene, storage stability for long periods, in terms, for example, of the maintenance of an emulsification state together with the maintenance of suppression of decomposition of the lycopene itself, is required.

The invention was made in view of such circumstances, and an object of the invention is to provide a lycopene-containing oil-in-water emulsion composition having excellent storage stability, and a method of producing the lycopene-containing oil-in-water emulsion composition.

Solution to Problem

Means for solving the problems described above are as follows.

[1] A lycopene-containing oil-in-water emulsion composition, including: an oil phase containing lycopene in an amount of from 0.00001% to 0.1% by mass or less with respect to a total mass of the emulsion composition, and an aqueous phase containing at least one selected from the group consisting of ascorbic acid, derivatives thereof, salts of ascorbic acid and an salts of derivatives of ascorbic acid, wherein an average particle diameter of emulsion particles is 120 nm or less.

[2] The lycopene-containing oil-in-water emulsion composition according to [1], including the lycopene in a non-crystalline state.

[3] The lycopene-containing oil-in-water emulsion composition according to [1] or [2], further including an emulsifier having an HLB of 10 or more in an amount of from 50% by mass to 2,000% by mass or less with respect to an amount of the oil phase and from 0.1% by mass to 15% by mass with respect to the total mass of the emulsion composition.

[4] The lycopene-containing oil-in-water emulsion composition according to [3], wherein the emulsifier having an HLB of 10 or more is a polyglycerin fatty acid ester.

[5] The lycopene-containing oil-in-water emulsion composition according to any one of [1] to [4], further including a fatty acid ester component having a total carbon number of 10 to 60 and having no hydroxyl group in the molecule.

[6] The lycopene-containing oil-in-water emulsion composition according to any one of [1] to [5], wherein the emulsion particles have an average particle diameter of from 5 nm to 100 nm or less.

[7] The lycopene-containing oil-in-water emulsion composition according to any one of [1] to [6], wherein the content of the lycopene is from 0.0002% by mass to 0.05% by mass with respect to the total mass of the emulsion composition.

[8] The lycopene-containing oil-in-water emulsion composition according to any one of [1] to [7], wherein the at least one selected from the group consisting of ascorbic acid, derivatives thereof, salts of ascorbic acid and salts of derivatives of ascorbic acid, is ascorbic acid, sodium ascorbate or a combination thereof.

[9] A method of producing the lycopene-containing oil-in-water emulsion composition according to any one of [1] to [8], the method including: preparing an oil-in-water emulsion composition containing lycopene; and adding at least one selected from the group consisting of ascorbic acid, derivatives thereof, salts of ascorbic acid and salts of derivatives of ascorbic acid to the oil-in-water emulsion composition.

Advantageous Effects of Invention

According to the invention, a lycopene-containing oil-in-water emulsion having excellent storage stability, and a method of producing the lycopene-containing oil-in-water emulsion are provided.

DESCRIPTION OF EMBODIMENTS

[Lycopene-containing oil-in-water emulsion composition]

The lycopene-containing oil-in-water emulsion composition of the invention (hereinafter, may be appropriately referred to as "emulsion composition of the invention") is an oil-in-water emulsion composition including an oil phase containing lycopene in an amount of from 0.00001% by mass to 0.1% by mass with respect to a total mass of the emulsion composition, and an aqueous phase containing at least one kind selected from the group consisting of ascorbic acid, derivatives thereof, salts of ascorbic acid and salts of derivatives of ascorbic acid (hereinafter, may be appropriately referred to as "ascorbic acid compound(s)"), wherein an average particle diameter of emulsion particles is 120 nm or less.

The emulsion composition of the invention exhibits excellent storage stability as a result of having the configuration described above.

Furthermore, in general, in an emulsion composition containing an oil-soluble component that is easily oxidized, the opportunity for contact with an oxidation active species increases as the particle diameter of the emulsion particles decreases, whereby the storage stability decreases. In contrast, it has been found in the emulsion composition of the invention that the suppression of decomposition of the lycopene and storage stability over a long time period of the emulsion composition are specifically improved by setting the particle diameter of the emulsion particles at a small particle size of 120 nm or less (more preferably 100 nm or less), setting the content of the lycopene contained in the emulsion composition to within a predetermined range, and incorporating at least one kind of compound selected from ascorbic acid compounds in the aqueous phase.

Furthermore, in general, a component that is easily oxidized is made unstable by being formed in a non-crystallized form since the opportunity for contact with an oxidation active species increases. In contrast, in a preferable embodiment of the emulsion composition of the invention, it was found that the stability is improved yet more significantly by a non-crystallized lycopene.

The emulsion composition of the invention is an oil-in-water emulsion composition, and is preferably obtained by mixing and emulsification of an aqueous phase composition composed of aqueous phase components and an oil phase composition composed of oil phase components.

In the invention, any numerical range expressed herein using "to" refers to a range including the numerical values before and after "to" as the minimum and maximum values, respectively.

In a case in which the amount of a component in the composition is indicated in the invention, when there are plural substances corresponding to the component in the composition, the indicated amount means the total amount of the plural substances present in the composition, unless specifically stated otherwise.

In the invention, the term "aqueous phase" is used as a term contrasting with "oil phase", regardless of the type of solvent.

In the specification, the term "process" encompasses an independent process, as well as a process that cannot be clearly distinguished from another process but yet achieves the expected effect of the process of interest.

In the specification, the expression "(poly)glycerin fatty acid ester" includes all glycerin fatty acid esters containing one glycerin unit and one fatty acid unit, a glycerin fatty acid ester containing plural units of either of the glycerin unit and the fatty acid unit, and a glycerin fatty acid ester containing plural units of both of the glycerin unit and the fatty acid unit. The expression "(poly)glycerin fatty acid ester" is used when using these glycerin fatty acid esters without distinction.

In the invention, the "dissolution temperature of lycopene" means the lowest temperature at which, when a lycopene crystalline body or a composition including a lycopene crystalline body is maintained for one minute at that temperature, the lycopene crystalline body completely dissolves.

Furthermore, in the invention, "storage stability" means that the decomposition of the lycopene that is included in the composition over time is suppressed, and the stability of the emulsification state in terms, for example, of the emulsion particle diameter is sustained without deteriorating, after the preparation of the emulsion composition.

Hereinbelow, the various constituent elements of the invention will be explained in detail.

<Lycopene to be contained in oil phase and relating matters>

The emulsion composition of the invention includes an oil phase containing a lycopene in which the content of the lycopene is from 0.00001% by mass to 0.1% by mass with respect to the total mass of the emulsion composition. The emulsion particles in the invention present as the oil phase (dispersion phase) in the emulsion composition of the invention, which is of an oil-in-water type.

The content of the lycopene in the emulsion composition of the invention is from 0.00001 % by mass to 0.1 % by mass, more preferably from 0.0002% by mass to 0.05% by mass, further preferably from 0.001% by mass to 0.04% by mass with respect to the total mass of the emulsion composition. The intended functions possessed by lycopene can be expected when the content of the lycopene in the emulsion composition is 0.00001% by mass or more, and excellent storage stability is exhibited when the content of lycopene in the emulsion composition is 0.1% by mass or less.

Furthermore, from the viewpoint of exhibiting the functions expected of lycopene, the content of lycopene in the emulsion particles is preferably from 0.1% by mass to 7% by mass, more preferably from 0.3% by mass to 5% by mass, and further preferably from 0.5% by mass to 4% by mass.

- Lycopene -

Lycopene is a carotenoid represented by the chemical formula: $C_{40}H_{56}$ (molecular weight 536.87), and is a red pigment which belongs to carotenes, which is a class of carotenoids, and exhibits an absorption maximum at 474 nm (acetone).

Lycopene is known to be excellent in an oxidation inhibitor effect, a skin whitening effect and the like, and addition thereof to the raw materials of foods, cosmetics and pharmaceuticals, and processed products thereof has been hitherto desired, investigated, and implemented.

Lycopene also has cis- and trans-isomers of the conjugated double bond at the center of the molecule, and examples thereof include an all-trans form, a 9-cis form, and a 13-cis form. According to the invention, any of these lycopene forms may be used.

Lycopene may also be used as a lycopene-containing oil or a lycopene-containing paste, which has been separated and extracted from a natural product containing lycopene, in the preparation of the emulsion composition of the invention.

Lycopene is contained in natural products such as tomato, persimmon, watermelon, and pink grapefruit, in nature. The lycopene-containing oil may be an oil that has been separated and extracted from these natural products.

Regarding the form of a product containing lycopene, there are known four kinds such as an oil type, an emulsion type, a paste type, and a powder type.

Furthermore, the lycopene used in the invention may be a product obtained by appropriately purifying an extract from a natural product as necessary. Furthermore, the lycopene used in the invention may also be a synthetic product.

One of the particularly preferred forms of lycopene may be lycopene derived from a tomato. Lycopene derived from a tomato includes an oil-soluble extract extracted from tomato pulp. The lycopene contained in an oil-soluble extract extracted from tomato pulp is particularly preferable from the viewpoints of stability, product quality, and productivity.

Here, an oil-soluble extract extracted from tomato pulp means an extract extracted from a pulp-like solid obtained by centrifuging a crushed product obtained by crushing tomatoes, using an oily solvent.

As the oil-soluble extract extracted from tomato pulp, tomato extracts that are widely sold in the market as lycopene-containing oils or lycopene-containing pastes can be used. Examples of tomato extracts that are commercially available include LYC-O-MATO 15% and LYC-O-MATO 6% available from Sunbright Co., Ltd.; and LYCOPENE 18 available from Kyowa Hakko Kogyo Bio Co., Ltd.

As a method for purifying a tomato-derived oil-soluble extract, the method shown below is exemplified.

An oil-soluble extract extracted from tomato pulp, which is available as a lycopene-containing oil or paste, is prepared. The oil-soluble extract is then dissolved in an oily solvent (for example, ethyl acetate or the like) to prepare a solution, ion-exchanged water is added to the solution and stirred, and the solution is separated into an oil phase liquid and an aqueous phase liquid. The obtained oil phase liquid is dried to give a purified product containing an oil-soluble component.

Carotinoid components other than lycopene may also be incorporated in the emulsion composition of the invention as long as the effect of the invention is not deteriorated. Specific examples of the carotinoid components other than lycopene include α-carotene, β-carotene, γ-carotene, δ-carotene, actinioerythrol, bixin, canthaxanthin, capsorubin, β-8'-apocarotenal (apocarotenal), β-12'-apocarotenal, xanthophylls (for example, astaxanthin, fucoxanthin, lutein, zeaxanthin, capsanthin, β-cryptoxanthin, violaxanthin and the like), and hydroxyl or carboxyl derivatives thereof. These other carotinoid components may be used singly or in combination of two or more kinds thereof.

Lycopene is a crystalline carotinoid, and is preferably contained in a non-crystalline state in the emulsion composition of the invention. When the lycopene is in a non-crystalline state, it is easier to adjust the average particle diameter of the emulsion particles to 120 nm or less. Furthermore, the lycopene in a non-crystalline state not only contributes to improving the storage stability of the lycopene in the emulsion composition of the invention, but also is capable of increasing the absorbability of lycopene by the body.

The lycopene contained in the invention is preferably lycopene in which 50% by mass to 100% by mass is a non-crystalline state, more preferably 90% by mass to 100% by mass is a non-crystalline state, and further preferably 95% by mass to 100% by mass is a non-crystalline state.

The question of whether or not lycopene is in a non-crystalline state and the content ratio of the lycopene in a non-crystalline state may be confirmed according to a conventional method, and differential scanning calorimetry (DSC), observation under a polarized-light microscope, X-ray diffractometry and the like can be utilized.

For example, this can be confirmed by comparing the amount of heat absorption of an endothermic peak originating from lycopene crystals in the composition of the invention measured by differential scanning calorimetry (DSC) with the amount of heat absorption of an endothermic peak of a lycopene crystal standard product.

Further, this can also be confirmed by comparing the spectrum of the emulsion composition of the invention in X-ray diffraction with the spectrum of a lycopene crystalline standard product. The lycopene can be deemed to be in a non-crystalline state when the detection of a crystal form cannot be confirmed by these known technologies.

In regard to whether or not a crystalline carotenoid is in a non-crystalline state, it is preferable to confirm whether or not at least one of the following is satisfied: that the endothermic and exothermic temperatures are determined, using a DSC Q2000 (TA Instruments Japan, Inc.), in one cycle of temperature increase-temperature decrease (15°C/min) in a temperature range of from 30°C to 200°C, in a state that moisture has been eliminated through freeze-drying, and the presence of a recognizable endotherm peak is not recognized; and that when an emulsion composition having a content of a crystalline carotenoid of 0.1% by mass is observed by polarization microscopic observation, there are 1000 or fewer recognizable crystalline bodies in a viewing field that measures 1 cm x 1 cm.

Further, the content ratio of lycopene can be calculated from a result obtained using a commercially available reagent as a crystalline body, and determining the content ratio from the DSC peak area or XRD (X-ray diffraction) based on the reagent being taken as 100%. Examples of the commercially available product of a reagent as a crystalline body include the reagents for biochemical applications available from Wako Pure Chemical Industries, Ltd.

The emulsion particles containing lycopene, which is contained in the emulsion composition of the invention, are emulsion particles having an average particle diameter of 120 nm or less.

The average particle diameter of the emulsion particles means the average particle diameter of the emulsion particles (oil droplets) that are present in the emulsion composition.

The average particle diameter of the emulsion particles in the invention is 120 nm or less, preferably 100 nm or less, more preferably 5 nm to 100 nm, further preferably from 10 nm to 100 nm, further preferably from 25 nm to 90 nm, further preferably from 40 nm to 80 nm, from the viewpoints of storage stability and transparency of the composition.

From the viewpoints of the particle diameter range and ease of measurement, the method of measuring the average particle diameter of the emulsion particles in the invention is preferably a dynamic light scattering method. Examples of commercially available analyzers utilizing dynamic light scattering include a NANOTRAC UPA (Nikkiso Co., Ltd.), a dynamic light scattering type particle size distribution analyzer, LB-550 (Horiba, Ltd.), and a concentrated system particle size analyzer, FPAR-1000 (Otsuka Electronics Co, Ltd.); however, for the particle size according to the invention, a value measured at 25°C using a particle size analyzer, FPAR-1000 (Otsuka Electronics Co., Ltd.) or NANOTRAC UPA (Nikkiso Co., Ltd.) is employed.

Specifically, the emulsion composition is diluted with pure water as necessary so as to obtain an oil phase concentration of 0.5% by mass, and the particle size is determined as the median diameter (d = 50) using a particle size analyzer "FPAR-1000 (Otsuka Electronics Co., Ltd.)". In the case when the oil phase concentration in the emulsion composition is 0.5% mass or less, the emulsion composition may be measured as a neat liquid without dilution. In the case when a stable measurement value cannot be obtained by the measurement by FPAR-1000 in the form of the original neat liquid, the particle diameter is obtained as a median diameter (d = 50) by measuring the original neat liquid using a NANOTRAC UPA (manufactured by NIKKISO CO., LTD.).

Furthermore, the average particle diameter of the emulsion particles can be adjusted by factors such as the stirring conditions in the production method (shear force, temperature and pressure) and the ratio of the oil phase to the aqueous phase besides the components in the composition.

Furthermore, in the emulsion composition of the invention, the content of the oil phase composition contained as the emulsion particles is preferably from 0.001% by mass to 30% by mass, more preferably from 0.005% by mass to 20% by mass, further preferably from 0.01% by mass to 10% by mass, from the viewpoint of exhibiting the functions of the oily components.

<Ascorbic acid compound(s) to be contained in aqueous phase and related matters thereof>

The emulsion composition of the invention contains at least one selected from the group consisting of ascorbic acid, derivatives thereof, salts of ascorbic acid and salts of derivatives of ascorbic acid (ascorbic acid compounds) in the aqueous phase.

The ascorbic acid compound may be used singly by one kind, or two or more kinds may be used in combination.

Ascorbic acid may be in any of L-form, D-form and DL-form, and the L-form is preferred from the viewpoint of availability and the like. A water-soluble ascorbic acid compound is preferable.

Examples of the ascorbic acid compounds includes sodium L-ascorbate, potassium L-ascorbate, calcium L-ascorbate, L-ascorbic acid phosphate ester, magnesium salt of L-ascorbic acid phosphate ester, L-ascorbic acid sulfate ester, disodium salt of L-ascorbic acid sulfate ester, L-ascorbic acid stearate ester, L-ascorbic acid 2-glucoside, L-ascorbyl palmitate ester, tetraisopalmitic acid L-ascorbyl and the like; fatty acid esters of ascorbic acid such as L-ascorbyl stearate ester, L-ascorbyl tetraisopalmitate ester and L-ascorbyl palmitate ester, and the like.

Among the group of ascorbic acid compounds described above, L-ascorbic acid, sodium L-ascorbate, calcium L-ascorbate, magnesium salt of L-ascorbic acid phosphate ester or disodium salt of L-ascorbic acid sulfate ester is preferable from the viewpoints of suppression of the decomposition and storage stability of lycopene.

Furthermore, as the ascorbic acid compound, a commercially available product may be suitably used.

Some particularly suitable embodiments of the ascorbic acid compound are ascorbic acid, sodium ascorbate or a combination thereof, from the viewpoint that a sufficient effect can be obtained even with a small amount.

In the invention, the total content of the ascorbic acid compound contained in the aqueous phase is preferably from 0.01% by mass to 5% by mass, more preferably from 0.03% by mass to 3% by mass, and further more preferably from 0.1% by mass to 1% by mass, with respect to the total mass of the emulsion composition, from the viewpoints of suppression of decomposition and the storage stability of lycopene. When the mass of the ascorbic acid compound is 0.01% by mass or more of the emulsion composition, this is sufficient to exhibit an effect of suppressing the reduction of lycopene content, whereas when the mass is 5% by mass or less, the particle size stability of the emulsion particles in the emulsion compositiondoes not deteriorate.

<(Poly)glycerin fatty acid ester>

The emulsion composition of the invention preferably contains a (poly)glycerin fatty acid ester as the oil phase component contained in the emulsion particles.

The other (poly)glycerin fatty acid ester is a (poly)glycerin fatty acid ester having a number of glycerin units of from 1 to 5, a number of fatty acid units of from 1 to 6, and at least one hydroxyl group of a glycerin unit.

In a co-dissolution product of such a specific (poly)glycerin fatty acid ester and lycopene, recrystallization of lycopene is suppressed.

A (poly)glycerin fatty acid ester having a number of glycerin unit(s) of 5 or less has high affinity with lycopene, and a (poly)glycerin fatty acid ester having a number of fatty acid unit(s) of 6 or less has a high effect of suppressing crystals of lycopene. Furthermore, by including a (poly)glycerin fatty acid ester containing a hydroxyl group(s) of a glycerin unit(s), a crystallization of lycopene can be sufficiently suppressed.

The (poly)glycerin fatty acid ester is preferably an ester between a glycerin having a number of glycerin units (average degree of polymerization) of from 1 to 5, and more preferably from 1 to 4, and a fatty acid having a number of fatty acid units of from 1 to 6, and more preferably from 1 to 5, and having a carbon number of from 8 to 22, and more preferably a fatty acid having a carbon number of from 14 to 18, from the viewpoint of suppressing recrystallization of lycopene, or the like. Examples of the fatty acid having a carbon number of from 8 to 22 include caprylic acid, capric acid, lauric acid, myristic acid, palmitic acid, stearic acid, oleic acid, linoleic acid, and behenic acid.

Among these other (poly)glycerin fatty acid esters, from the viewpoint of uniform solubility at the time of co-dissolution, a (poly)glycerin fatty acid ester having a molecular weight of 10,000 or less is preferred, a (poly)glycerin fatty acid ester having a molecular weight of 3,000 or less is more preferred, and a (poly)glycerin fatty acid ester having a molecular weight of 2,500 or less is still more preferred. Furthermore, from the viewpoint of affinity with lycopene, a (poly)glycerin fatty acid ester having a HLB value of 9 or less is preferred, and a (poly)glycerin fatty acid ester having a HLB value of 6 or less is more preferred.

Examples of the (poly)glycerin fatty acid ester that can be used in the emulsion composition of the invention include glyceryl myristate, glyceryl monostearate, diglyceryl monostearate, triglyceryl monostearate, pentaglyceryl monostearate, triglyceryl dipalmitate, glyceryl distearate, tetraglyceryl tristearate, tetraglyceryl pentastearate, hexaglyceryl tetrabehenate, and the like. From the viewpoints of suppression of recrystallization and uniform solubility, glyceryl myristate, glyceryl monostearate, diglyceryl monostearate, tetraglyceryl pentastearate, or tetraglyceryl tristearate is preferred.

The content (mass) of the other (poly)glycerin fatty acid ester is preferably from 0.01 times to 9 times, more preferably from 0.1 times to 8 times, and still more preferably from 0.3 times to 5 times, with respect to the total mass of lycopene from the viewpoint of stability of the emulsion composition.

When the total mass of the (poly)glycerin fatty acid ester in the emulsion composition is 0.01 times the total mass of the lycopene, a sufficient crystal suppressing effect can be expected, while when the total mass is 9 times or less, an increase in the particle diameter of the emulsion particles obtainable when an emulsion is produced, can be suppressed.

<Other fatty acid esters>

It is preferable that the emulsion composition of the invention contains a triester of glycerin and an fattyc acid, and an ester of an alcohol having one hydroxyl group and an fatty acid (hereinafter, also referred to as "other fatty acid ester").

The preferred other fatty acid ester component is a fatty acid ester component having a total carbon number of from 10 to 60 and having no hydroxyl group in the molecule, which is at least one selected from the group consisting of a triester of glycerin and a fatty acid, and an ester of an alcohol having one hydroxyl group and a fatty acid.

Such a specifc fatty acid ester component has a higher effect of decreasing the dissolution temperature of lycopene than that of the above-mentioned (poly)glycerin fatty acid ester containing the hydroxyl group(s) of the glycerin unit(s), the thermal decomposition during the amorphization of the lycopene by heating can be suppressed. Further, due to the fatty acid ester component, the fineness of the emulsion particles in the invention can be retained more stably.

When the total carbon number in the other fatty acid ester is 10 or more, the increase in the particle diameter of the emulsion particles tends to be further suppressed. Further, when the total carbon number is 60 or less, there is a tendency that the dissolution temperature of the crystalline carotenoid is sufficiently lowered.

From the viewpoint of lowering the dissolution temperature of a crystalline carotenoid such as lycopene, the other fatty acid ester preferably has a total carbon number of from 10 to 60, and more preferably has a total carbon number of from 27 to 57.

Further, each of the fatty acid units in the other fatty acid ester is preferably a fatty acid unit having a carbon number of from 8 to 18, more preferably a fatty acid unit having a carbon number of from 8 to 12, and still more preferably a fatty acid unit having a carbon number of from 8 to 10, from the viewpoint of suppressing a increase in the particle diameter of the emulsion particles.

The triester of glycerin and a fatty acid preferably has a total carbon number of from 10 to 60, and more preferably has a total carbon number of from 27 to 57, from the viewpoint of lowering the dissolution temperature of the crystalline carotenoid such as lycopene.

Each of the three fatty acid units in the triester of glycerin and a fatty acid is preferably a fatty acid unit having a carbon number of from 8 to 18, more preferably a fatty acid unit having a carbon number of from 8 to 12, and still more preferably a fatty acid unit having a carbon number of from 8 to 10, from the viewpoint of suppressing an increase in the particle diameter of the emulsion particles in the emulsion composition. The fatty acid unit may be a fatty acid unit derived from a saturated fatty acid, or may be a fatty acid derived from an unsaturated fatty acid. Also, the fatty acid unit may be a fatty acid unit derived from a straight-chained fatty acid, or may be a fatty acid unit derived from a branched fatty acid. Among them, from the viewpoint of lowering the dissolution temperature of a crystalline carotenoid such as lycopene, the fatty acid unit is preferably a fatty acid unit derived from a straight-chained fatty acid.

Specific examples of the triester of glycerin and a fatty acid include glyceryl tricaprylate, glyceryl tricaprate, glyceryl trilaurate, glyceryl trimyristate, glyceryl tripalmitate, glyceryl tripalmitoleate, glyceryl tristearate, glyceryl trioleate, glyceryl trilinolate, glyceryl trilinolenate, and glyceryl tri(caprylate/caprate).

From the viewpoint of lowering the dissolution temperature of the crystalline carotenoid such as lycopene, glyceryl tricaprylate, glyceryl tricaprate, glyceryl trilaurate, glyceryl tri(caprylate/caprate), and the like are preferred.

The triester of glycerin and a fatty acid may be used singly, or may be used in combination of two or more kinds thereof.

Furthermore, olive oil, camellia oil, macadamia nut oil, castor oil, avocado oil, evening primrose oil, turtle oil, corn oil, mink oil, rapeseed oil, egg yolk oil, sesame oil, persic oil, wheat germ oil, sasanqua oil, linseed oil, cotton seed oil, perila oil, soybean oil, tea seed oil, Japanese nutmeg oil, rice bran oil, Chinese tung oil, Japanese tung oil, jojoba oil, germ oil, glycerin trioctanoate, glycerin triisopalmitate, salad oil, safflower oil, coconut oil, peanut oil, almond oil, hazelnut oil, walnut oil, grape seed oil, cacao fat, palm oil, shortening, and the like, which are mixtures of trimesters of glycerin and fatty acids, can also be used.

In regard to the ester of an alcohol having one hydroxyl group and a fatty acid, an ester having a total carbon number of from 10 to 50 is preferred, and an ester having a total carbon number of from 10 to 30 is more preferred, from the viewpoint of suppressing an increase in the particle diameter of the emulsion particles.

In regard to the fatty acid unit for the ester of an alcohol having one hydroxyl group and a fatty acid, from the viewpoint of lowering the dissolution temperature of a crystalline carotenoid such as lycopene, a fatty acid unit having a carbon number of from 8 to 18 is preferred, a fatty acid unit having a carbon number of from 8 to 12 is more preferred, and a fatty acid unit having a carbon number of from 8 to 10 is still more preferred. The fatty acid unit may be a fatty acid unit derived from a saturated fatty acid, or may be a fatty acid unit derived from an unsaturated fatty acid. Furthermore, the fatty acid unit may be a fatty acid unit derived from a straight-chained fatty acid, or may be a fatty acid unit derived from a branched fatty acid. Among them, from the viewpoint of lowering the dissolution temperature of the crystalline carotenoid such as lycopene, the fatty acid unit is preferably a fatty acid unit derived from a straight-chained fatty acid.

In regard to the alcohol unit for the ester of an alcohol having one hydroxyl group and a fatty acid, an alcohol unit having a carbon number of from 2 to 35 is preferred, an alcohol unit having a carbon number of from 4 to 20 is more preferred, and an alcohol unit having a carbon number of from 5 to 15 is still more preferred, from the viewpoint of lowering the dissolution temperature of lycopene. The alcohol unit may be an alcohol unit derived from a saturated alcohol, or may be an alcohol unit derived from an unsaturated alcohol. Furthermore, the alcohol unit may be an alcohol unit derived from a straight-chained alcohol, or may be an alcohol unit derived from a branched alcohol. Among them, from the viewpoint of lowering the dissolution temperature of lycopene, the alcohol unit is preferably an alcohol unit derived from a straight-chained alcohol.

Examples of the ester of an alcohol having one hydroxyl group and a fatty acid include hexyl caprylate, hexyl laurate, methyl heptyl laurate, octyl dodecyl myristate, methyl heptyl isostearate, isocetyl isostearate, methyl heptyl isostearate, isopropyl isostearate, butyl stearate, 2-ethylhexyl stearate, and the like. From the viewpoint of lowering the dissolution temperature of lycopene, methyl heptyl laurate, methyl heptyl isostearate, and the like are preferred.

The ester of an alcohol having one hydroxyl group and a fatty acid may be used singly, or in combination of two or more kinds thereof.

Furthermore, regarding the fatty acid ester component, two or more kinds may be used in combination, irrespective of the kinds of the triester of glycerin and a fatty acid, and of the ester of an alcohol having one hydroxyl group and a fatty acid.

The content (mass) of the other fatty acid ester is preferably from 3-fold amount to 300-fold amount, more preferably from 5-fold amount to 200-fold amount, and further preferably from 7-fold amount to 100-fold amount, in view of decreasing of the solution temperature of the lycopene in the emulsion composition.

If the total mass of the other fatty acid ester component in the emulsion composition is 3-fold amount or more of the total mass of the lycopene, a sufficient crystal suppression effect can be expected. On the other hand, if the total mass is 300-fold amount or less, incorporation of a sufficient amount of lycopene is not deteriorated. The content (mass) of the other fatty acid ester is preferably from 3 times to 300 times, more preferably from 5 times to 200 times, and still more preferably from 7 times to 100 times, the total mass of lycopene, from the viewpoint of lowering the dissolution temperature of lycopene in the emulsion composition.

When the total mass of the other fatty acid ester component in the emulsion composition of the invention is 3 times or more the total mass of lycopene, a sufficient effect of lowering the dissolution temperature of lycopene can be expected. On the other hand, when the content is 300 times or less, incorporation of a sufficient amount of lycopene can be achieved without impairment.

The content of the other fatty acid ester may vary with the kind or content of the above-mentioned (poly)glycerin fatty acid ester used; however, from the viewpoint of stability of the emulsion composition, the content of the other fatty acid ester is preferably from 0.8 times to 750 times, more preferably from 1 time to 300 times, and still more preferably from 2 times to 100 times, with respect to the total mass of the above-mentioned (poly)glycerin fatty acid ester.

When the total mass of the other fatty acid ester in the emulsion composition is 0.8 times the total mass of the above-mentioned (poly)glycerin fatty acid ester, a sufficient effect of enhancing the stability of a carotenoid-containing composition can be expected. On the other hand, when the total mass of the other fatty acid ester is 750 times or less the total mass of the above-mentioned (poly)glycerin fatty acid ester, incorporation of a sufficient amount of the carotenoid can be achieved without impairment.

It is preferable, from the viewpoint of suppression of crystallization of lycopene and stability, that the other fatty acid ester is a triester of glycerin and a fatty acid, the content (mass) of the other fatty acid ester is from 7 times to 100 times the amount of lycopene, the total mass of the above-mentioned (poly)glycerin fatty acid ester is from 0.3 times to 5 times the total mass of lycopene, and the content (mass) of the other fatty acid ester is from 2 times to 100 times the total mass of the above-mentioned (poly)glycerin fatty acid ester.

<Other oily component>

As the component that constitutes the emulsion particles in the emulsion composition of the invention, the emulsion composition may further include other oily components besides the various components previously mentioned above.

Regarding the other oily components, any component which has a solubility in water at 25°C of less than 0.5% by mass, and dissolves in the oil phase components including the lycopene according to the invention at 90°C in an amount of 5% by mass or more, may be used without any particular limitations, and any such compound having properties or functionality according to the purpose can be appropriately selected and used. For example, an oxidation inhibitor, a non-cryatalline-carotenoid, unsaturated fatty acids, squalane, squalene and the like are preferably used.

Examples of the unsaturated fatty acid include monovalent highly-unsaturated aliphatic acids ($\omega$-9, oleic acid and the like) or polyvalent highly-unsaturated aliphatic acids ($\omega$-3, $\omega$-6) each having a carbon number of 10 or more, preferably 18 to 30. Such unsaturated fatty acids may be any of known ones, and examples of $\omega$-3 oils or fats can include linolenic acid, eicosapentaenoic acid (EPA) and docosapentaenoic acid (DHA), and fish oils containing these oils or fats, and the like.

Examples of ubiquinones include Coenzyme Qs such as Coenzyme Q10, and the like.

Furthermore, fatty acid esters of erythorbic acid such as palmitic acid erythorbyl ester and tetraisopalmitic acid erythorbyl ester; fatty acid esters of Vitamin $B_6$ such as pyridoxine dipalmitate, pyridoxine tripalmitate, pyridoxine dilaurylate and pyridoxine dioctanoate, and the like can also be exemplified as oil-soluble vitamins.

<Oxidation inhibitor>

The emulsion composition of the invention preferrably contains an oxidation inhibitor in the oil phase. One kind of oxidation inhibitor may be used singly, or two or more kinds of oxidation inhibitors may be used in combination. It is presumed that the emulsion composition of the invention enables sure suppression of the decomposition (for example, oxidation decomposition or the like) of the lycopene by heating, by containing the above-mentioned oxidation inhibitor.

As the oxidation inhibitor contained in the oil phase, for example, among the various antioxidants described in "Kosanka-zai no Riron to Jissai (Theory and Practice of Antioxidants)" (written by Kajimoto, Daisan Shobo, 1984), and the various oxidation inhibitors described in "Sanka Boshizai Handobuku (Handbook of Oxidation Inhibitors)" (written by Saruwatari, Nishino, and Tabata, Taiseisha, 1976), those functioning as oxidation inhibitors may be used. Specifically, the oxidation inhibitor is preferably at least one selected from the group consisting of compounds having a phenolic hydroxyl group.

Preferable oxidation inhibitors will be exemplified below, but the invention is not limited by these oxidation inhibitors.

One of the preferable oxidation inhibitors for the emulsion composition of the invention is a phenolic oxidation inhibitor. It is thought that the emulsion composition of the invention enables sure suppression of the decomposition (for example, oxidation decomposition or the like) of the crystalline carotenoid by heating, by containing the phenolic oxidation inhibitor. The phenolic oxidation inhibitor as used herein refers a compound that acts as an oxidation inhibitor among compounds each having a phenolic hydroxyl group.

Examples of the phenolic oxidation inhibitor include aromatic carboxylic acid compounds, cinnamic acid compounds or ellagic acid compounds, BHT (butylhydroxytoluene), BHA (butylhydroxyanisol), Vitamin E compounds and the like.

Examples of the aromatic carboxylic acid compounds include gallic acid (3,4,5-hydroxybenzoic acid) and derivatives thereof. Examples of the derivatives of gallic acid (3,4,5-hydroxybenzoic acid) include gallic acid esters such as propyl gallate, epicatechin gallate and epigallocatechin gallate, and gallic acid glycosides such as gallotannin.

Examples of the cinnamic acid compounds include ferulic acid and chlorogenic acid and the like, and derivatives thereof. Examples of the derivatives of ferulic acid and chlorogenic acid include ferulic acid esters. Specific examples include ferulic acid, $\gamma$-oryzanol (rice bran extractive matter), coffee acid (caffeic acid or 3,4-dihydroxycinnamic acid), chlorogenic acid, glycerylferulic acid, dihydroferulic acid, and the like.

Examples of the ellagic acid compounds include ellagic acid.

Vitamin E compounds are not particularly limited, and examples thereof include those selected from a compound group including tocopherol and derivatives thereof, and a compound group including tocotrienol and derivatives thereof. These vitamin E compounds may be used singly, or in combination of plural kinds thereof. Also, a compound selected respectively from the compound group including tocopherol and derivatives thereof and the compound group including tocotrienol and derivatives thereof may be used in combination.

The compound group consisting of tocopherol and derivatives thereof include dl-$\alpha$-tocopherol, dl-$\beta$-tocopherol, dl-$\gamma$-tocopherol, dl-$\delta$-tocopherol, dl-$\alpha$-tocopherol acetate, dl-$\alpha$-tocopherol nicotinate, dl-$\alpha$-tocopherol linolate, and dl-$\alpha$-tocopherol succinate. Among these, dl-$\alpha$-tocopherol, dl-$\beta$-tocopherol, dl-$\gamma$-tocopherol, dl-$\delta$-tocopherol, and mixtures thereof (mixed tocopherols) are more preferred. Furthermore, regarding the tocopherol derivatives, carboxylic acid esters, particularly acetic acid esters, of these tocopherols are preferably used.

The compound group including tocotrienol and derivatives thereof include $\alpha$-tocotrienol, $\beta$-tocotrienol, $\gamma$-tocotrienol, and $\delta$-tocotrienol. Furthermore, regarding the tocotrienol derivatives, acetic acid esters of these tocotrienols are preferably used.

As the phenolic oxidation inhibitor, cinnamic acid compounds (for example, ferulic acid, $\gamma$-orizanol, and mixtures thereof), or vitamin E compounds (for example, a compound group including tocopherol and derivatives thereof) are preferred from the viewpoint of the stability of lycopene, and among these, at least one selected from a compound group consisting of tocopherol, tocotrienol, and derivatives thereof is more preferred.

The molecular weight of the phenolic oxidation inhibitor is preferably a low molecular weight from the viewpoint of the stability of lycopene. As the compound having a phenolic hydroxyl group, for example, a compound having a molecular weight of from 100 to 3,000 is preferred, and a compound having a molecular weight of from 100 to 1,000 is more preferred.

The total content of the phenolic oxidation inhibitor in the emulsion composition of the invention may any amount effective for suppressing decomposition or loss of lycopene, and the total content can be adjusted to from 1.3 times to 15.0 times the moles of lycopene.

It is preferable to adjust the total content to from 2 times to 10 times, and more preferably from 3 times to 8 times, the molar amount. When the total content of the phenolic oxidation inhibitor is 1.3 or more times the moles of lycopene, the amount is sufficient for manifesting the effect of suppressing a decrease in the decomposition or loss of lycopene. When the total content is 15.0 or less times the moles of lycopene, incorporation of a sufficient amount of lycopene can be achieved without impairment.

Furthermore, the phenolic oxidation inhibitor is preferably contained in the oil phase composition that is formed from the oil phase component. In this case, it is also preferable to use both phenolic oxidation inhibitor(s) and ascorbic acid compound(s) in the oil phase composition. It is preferable to use both phenolic oxidation inhibitor(s) and ascorbic acid compound(s) since decomposition of the lycopene by heating (for example, oxidation decomposition or the like) can be reliably suppressed, and reduction of the lycopene in the production processes of the emulsion composition can be suppressed.

As the oxidant contained in the oil phase, an ascorbic acid compound can be used.

As the ascorbic acid compound, the compounds that are mentioned above as the compound contained in the aqueous phase can be used.

However, since there are some cases where a decomposed product of the ascorbic acid compound interferes with the particle size stability of the emulsion particles, it is preferable that an ascorbic acid compound is not used as far as possible.

<Emulsifier>

The emulsion composition of the invention may also contain an emulsifier that can be used as the oil phase component besides the above-mentioned components. Examples of such oil phase component include emulsifiers each having an HLB of 7 or less among the emulsifiers mentioned below.

Furthermore, the aqueous phase composition is an aqueous phase composition being constituted by an aqueous medium, especially water, and preferably containing at least an emulsifier.

The emulsifier may be any of an anionic surfactant, a cationic surfactant, an amphoteric surfactant and a nonionic surfactant.

Further, from the viewpoint of emulsifying power, the emulsifier is preferably one having an HLB value of 10 or more, and more preferably one having an HLB value of 12 or more. If the HLB value is too low, the emulsifying power may be sufficient. In addition, from the viewpoint of a defoaming effect, an emulsifier having a HLB value of from 5 or more but less than 10 may be used in combination.

Here, the HLB is the hydrophilicity-hydrophobicity balance that is conventionally used in the field of surfactants, and calculation formulas that are conventionally used, for example, Kawakami's formula, can be used. The Kawakami's formula is shown below.

$$HLB = 7 + 11.7\log(M_w/M_o)$$

Here, $M_w$ represents the molecular weight of a hydrophilic group, and $M_o$ represents the molecular weight of a hydrophobic group.

Furthermore, the values of HLB described in catalogues and the like may also be used.

As can be seen from the formula described above, an emulsifier having an arbitrary HLB value can be obtained by using the additivity of the HLB.

The content of the emulsifier in adjusting the particle diameter of the emulsion particles to 120 nm or less (preferably 100 nm or less) by conducting an emulsification operation in the preparation of the emulsion composition of the invention is preferably 0.5 times of or more, more preferably 0.55 times or more, and further preferably 0.6 times of or more from the viewpoints of the miniaturization and dispersion stability of the emulsified particles.

Furthermore, the content of the emulsifier is preferably 15% by mass or less, more preferably 10% by mass or less, further preferably 5% by mass or less with respect to the total mass of the emulsion composition, from the viewpoint of suppression of the bubbling of the emulsion composition.

Among the emulsifiers, nonionic surfactants are preferable since they have low stimulus property, affect little on the environments, and the like. Examples of the nonionic surfactants include a sucrose fatty acid ester, a polyglycerin fatty acid ester, an organic acid monoglyceride, a propylene glycol fatty acid ester, a polyglycerin condensed ricinoleic acid ester, a sorbitan fatty acid ester, a polyoxyethylene sorbitan fatty acid ester and the like. Among these, a polyglycerin fatty acid ester is especially preferable from the viewpoint of the miniaturizaion of the emulsion particles.

One preferable embodiment of the emulsion composition of the invention is an embodiment containing an emulsifier having an HLB of 10 or more in an amount of from 50% by mass to 2,000% by mass with respect to the amount of the oil phase, and from 0.1% by mass to 15% by mass with respect to the total mass of the emulsion composition. The emulsifier having an HLB of 10 or more is preferably a polyglycerin fatty acid ester.

The sucrose fatty acid ester is such that, from the viewpoint of stability of the dispersed particles in the composition, the carbon number of the fatty acid that constitutes the sucrose fatty acid ester is preferably from 12 to 20, and more preferably from 14 to 16.

Preferred examples of the sucrose fatty acid ester include sucrose dioleic acid ester, sucrose distearic acid ester, sucrose dipalmitic acid ester, sucrose dimyristic acid ester, sucrose dilauric acid ester, sucrose monooleic acid ester, sucrose monostearic acid ester, sucrose monopalmitic acid ester, sucrose monomyristic acid ester, and sucrose monolauric acid ester. Among these, sucrose monooleic acid ester, sucrose monostearic acid ester, sucrose monopalmitic acid ester, sucrose monomyristic acid ester, and sucrose monolauric acid ester are more preferred.

In the invention, these sucrose fatty acid esters can be used singly or in combination thereof.

The aqueous phase composition may contain a polyglycerin fatty acid ester as an emulsifier besides the above-mentioned polyglycerin fatty acid ester.

The polyglycerin fatty acid ester contained as such emulsifier is an ester of a polyglycerin having an average degree of polymerization of 6 or more, preferably from 6 to 15, more preferably from 8 to 10 and an fatty acid having a carbon number of from 8 to 18 such as capric acid, caprylic acid, lauryl acid, myristic acid, palmitic acid, stearic acid, oleic acid and linoleic acid.

Preferable examples of the polyglycerin fatty acid ester contained as an emulsifier include hexaglycerin monooleic acid ester, hexaglycerin monostearic acid ester, hexaglycerin monopalmitic acid ester, hexaglycerin monomyristic acid ester, hexaglycerin monolauryl acid ester, decaglycerin monooleic acid ester, decaglycerin monostearic acid ester, decaglycerin monopalmitic acid ester, decaglycerin monomyristic acid ester, decaglycerin monolauryl acid ester and the like.

Among these, decaglycerin monooleic acid ester (HLB = 12), decaglycerin monostearic acid ester (HLB = 12), decaglycerin monopalmitic acid ester (HLB = 13), decaglycerin monomyristic acid ester (HLB = 14), decaglycerin monolauryl acid ester (HLB = 16) and the like are more preferable.

These polyglycerin fatty acid esters can be used singly or by mixing.

Preferred examples of the polyglycerin fatty acid ester that is included as a surfactant include hexaglycerin monooleic acid ester, hexaglycerin monostearic acid ester, hexaglycerin monopalmitic acid ester, hexaglycerin monomyristic acid ester, hexaglycerin monolauric acid ester, decaglycerin monooleic acid ester, decaglycerin monostearic acid ester, decaglycerin monopalmitic acid ester, decaglycerin monomyristic acid ester, and decaglycerin monolauric acid ester.

Among these, more preferred are decaglycerin monooleic acid ester (HLB = 12), decaglycerin monostearic acid ester (HLB = 12), decaglycerin monopalmitic acid ester (HLB = 13), decaglycerin monomyristic acid ester (HLB = 14), and decaglycerin monolauric acid ester (HLB = 16), and the like.

These polyglycerin fatty acid esters can be used singly or in combination.

As the sorbitan fatty acid ester, such a compound having a carbon number of the fatty acid of 8 or more is preferred, and a compound having a carbon number of the fatty acid of 12 or more is more preferred. Preferred examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, sorbitan trioleate, and the like.

In the invention, these sorbitan fatty acid esters can be used singly or in combination.

As the polyoxyethylene sorbitan fatty acid ester, such a compound having a carbon number of the fatty acid of 8 or more is preferred, and a compound having a carbon number of the fatty acid of 12 or more is more preferred. The length of ethylene oxides (number of added moles) of the polyoxyethylene is preferably from 2 to 100, and more preferably from 4 to 50.

Preferred examples of the polyoxyethylene sorbitan fatty acid ester include polyoxyethylene sorbitan monocaprylate, polyoxyethylene sorbitan monolaurate, polyoxyethylene sorbitan monostearate, polyoxyethylene sorbitan sesquistearate, polyoxyethylene sorbitan tristearate, polyoxyethylene sorbitan isostearate, polyoxyethylene sorbitan sesquiisostearate, polyoxyethylene sorbitan oleate, polyoxyethylene sorbitan sesquioleate, polyoxyethylene sorbitan trioleate, and the like.

In the invention, these polyoxyethylene sorbitan fatty acid esters can be used singly or in combination.

Furthermore, the invention may also include a phospholipid such as lecithin as the emulsifier.

The phospholipid that can be used in the invention is a compound which contains a glycerin skeleton, a fatty acid residue, and a phosphoric acid residue as essential constituent components, and has a base, a polyhydric alcohol or the like bonded thereto. The phospholipid is also called lecithin. Since a phospholipid has a hydrophilic group and a hydrophobic group in the molecule, phospholipids have been widely used hitherto as emulsifiers in the fields of foods, cosmetics, and pharmaceuticals.

Industrially, a product having a lecithin purity of 60% or more is utilized as lecithin, and that can also be utilized in this invention. From the viewpoints of the formation of fine oil droplet particle diameter and the stability of functional oily components, preferred lecithin is what is generally referred to as high purity lecithin, and this is a product having a lecithin purity of 80% or more, and more preferably 90% or more..

Examples of the phospholipid include various conventionally known phospholipids that are extracted and separated from living organisms such as plants, animals, or microorganisms.

Specific examples of such phospholipids include various lecithins derived from plants such as soybean, corn, peanut, rapeseed, and wheat; animals such as egg yolk and beef; and microorganisms such as Escherichia coli.

Examples of such lecithin include, expressed in their compound names, glycerolecithins such as phosphatidic acid, phosphatidylglycerin, phosphatidylinositol, phosphatidylethanolamine, phosphatidylmethylethanolamine, phosphatidylcholine, phosphatidylserine, bisphosphatidic acid, and diphosphatidylglycerin (cardiolipin); and sphingolecithins such as sphingomyelin.

Also, according to the invention, hydrogenated lecithin, enzymatically degraded lecithin, enzymatically degraded hydrogenated lecithin, hydroxylecithin, and the like can also be used in addition to the high purity lecithin. These lecithins that can be used in the invention can be used singly, or in mixture of plural kinds thereof.

<Other additional components>

In addition to the various components described above, components that are conventionally used in the fields of foods, cosmetics and the like may be appropriately incorporated into the emulsion composition of the invention according to the form of the relevant composition. The additive components may be incorporated as the components of the oil phase composition or the components of the aqueous phase composition, depending on the characteristics of the additive components, or may be incorporated as additive components to the aqueous phase after the production of the emulsion composition.

Examples of such other components include polyhydric alcohols such as glycerin and 1,3-butylene glycol; monosaccharides or polysaccharides such as glucose, fructose, lactose, maltose, sucrose, pectin, kappa-carrageenan, locust bean gum, guar gum, hydroxypropyl guar gum, xanthan gum, karaya gum, tamarind seed polysaccharides, gum arabic, tragacanth gum, hyaluronic acid, sodium hyaluronate, sodium chondroitin sulfate, and dextrin; sugar alcohols such as sorbitol, mannitol, maltitol, lactose, maltotriitol, and xylytol; inorganic salts such as sodium chloride and sodium sulfate; proteins having molecular weights of more than 5000, such as casein, albumin, methylated collagen, hydrolyzed collagen, water-soluble collagen, and gelatin; synthetic polymers such as carboxyvinyl polymers, polysodium acrylate, polyvinyl alcohol, polyethylene glycol, and ethylene oxide-propylene oxide block copolymers; water-soluble cellulose derivatives such as hydroxyethyl cellulose and methyl cellulose; flavonoids (catechin, anthocyanin, flavones, isoflavone, flavane, flavanone, and rutin), lignans, curcumins, and coumarins. These additive components may be include in the emulsion composition as, for example, functional components, excipients, viscosity adjusting agents, and radical scavengers, based on their functions.

In addition to them, for example, various efficacious components, a pH adjusting agent, a pH buffering agent, an ultraviolet absorber, an antiseptic agent, a fragrance, and a colorant, that is, other additives that are conventionally used for those applications can be used in combination.

The emulsion composition of the invention is an emulsion composition from which a desired effect induced by lycopene is sufficiently expected since decomposition of lycopene is suppressed, and storage stability is excellent. Therefore, the emulsion composition of the invention can be preferably applied to food compositions, cosmetic compositions, pharmaceutical compositions, and the like.

Furthermore, components that can be added to foods or cosmetics can be appropriately added, if necessary, to foods or cosmetics that contain the emulsion composition of the invention.

Foods, cosmetics and the like that contain the emulsion composition of the invention may exhibit an effect that may not be sufficiently exhibited due to decomposition of lycopene or deterioration of an emulsion state, for example, satisfactory absorbability of lycopene.

The emulsion composition of the invention is suitably used in, for example, skin toners, essences, emulsions, cream packs/masks, packs, cosmetics for hair cleaning, fragrance cosmetics, liquid body cleanser, UV care cosmetics, deodorant cosmetics, oral care cosmetics, and the like.

Also, the emulsion composition of the invention is also suitably used in, as foods, general food products such as nutritional drinks, revitalizers, palatable beverages, and frozen desserts; as well as in nutrition supplement foods in capsule forms, and the like.

The emulsion composition of the invention can be produced by known methods. Regarding a preferred production method of the emulsion composition of the invention, the production method described below may be used.

[Method of producing emulsion composition]

The method of producing the emulsion composition of the invention includes mixing an oil phase composition containing lycopene with an aqueous phase composition, and emulsifying the mixture under pressure.

The addition of ascorbic acid compound(s) to the aqueous phase in the method of producing the emulsion composition of the invention may constitute an embodiment in which the ascorbic acid compound(s) is/are added to the aqueous phase composition before being mixed with the oil phase composition, or may constitute an embodiment in which the oil phase composition and the aqueous phase composition are mixed, the mixture is subjected to emulsification under pressure to prepare an emulsion composition, and the ascorbic acid compound(s) is/are then added to the emulsion composition, and the latter embodiment of addition is more preferable.

A preferable embodiment for obtaining an oil phase composition containing lycopene is an embodiment including heating the oil phase composition before it is mixed with the aqueous phase composition as mentioned below at a temperature condition of 90°C or more. In a case in which the oil phase composition is obtained by such an embodiment, considering the thermal decomposition properties shown by the ascorbic acid compound(s), an embodiment in which an emulsion composition containing lycopene is prepared, and ascorbic acid compound(s) is/are then added to the oil-in-water emulsion composition, is preferable.

The ascorbic acid compound(s) itself/themselves may be directly added to the aqueous phase composition or the prepared emulsion composition, and it is more preferable to prepare an aqueous solution containing the ascorbic acid compound, and add the aqueous solution to the aqueous phase composition or the prepared emulsion composition. If desired, optional additives may also be added to the aqueous solution.

One preferable method of producing the emulsion composition of the invention is a method including preparing an emulsion composition containing lycopene at a higher content than the lycopene content in the emulsion composition of the invention, and adding an aqueous solution containing an ascorbic acid compound using a diluted liquid to thereby adjust the lycopene content to a predetermined amount.

The content of the lycopene in the emulsion composition to which the aqueous solution containing the ascorbic acid compound is added is preferably from 0.001% by mass to 2% by mass, more preferably from 0.05% by mass to 1% by mass, further preferably from 0.1% by mass to 0.6% by mass, with respect to the total mass of the emulsion composition.

A preferable method of producing the emulsion composition to which the ascorbic acid compound is added in the invention will further be explained in detail.

According to a preferred embodiment for obtaining an oil phase composition containing lycopene, an embodiment is available that includesheating, under temperature conditions of 90°C or higher, an oil phase component mixed liquid including lycopene; a fatty acid ester component having a total carbon number of from 10 to 60 and having no hydroxyl group in the molecule, which is at least one selected from the group consisting of a triester of glycerin and a fatty acid, and an ester of an alcohol having one hydroxyl group and a fatty acid; and an oxidation inhibitor.

Hereinafter, the method of preparing an emulsion composition will be described in detail with reference to an example of the method of preparing an emulsion composition using the oil phase composition of the present embodiment, but the invention is not intended to be limited to this.

According to the production method of the present embodiment, since lycopene is heated together with a predetermined (poly)glycerin fatty acid ester, a predetermined fatty acid ester component, and an oxidation inhibitor under conditions at a temperature higher than or equal to the dissolution temperature of the carotenoid, lycopene co-dissoluves with the predetermined (poly)glycerin fatty acid ester and the predetermined fatty acid ester component. When this lycopene-containing oil phase composition obtained by co-dissolution is used as the oil phase composition that is to be heated and emulsified together with an aqueous phase composition containing a water-soluble emulsifier, the emulsion composition obtained by emulsification becomes a composition in which crystallization of lycopene, which is a crystalline carotenoid, is suppressed. The production method of the present embodiment constitutes addition of the predetermined fatty acid ester components. Thereby, it is possible to lower the dissolution temperature of lycopene, as compared with a case in which the predetermined fatty acid ester components are not added.

According to the production method of the present embodiment, first, lycopene; a fatty acid ester component having a total carbon number of from 10 to 60 and having no hydroxyl group in the molecule, which is at least one selected from the group consisting of a triester of glycerin and a fatty acid, and an ester of an alcohol having one hydroxyl group and a fatty acid; and an oxidation inhibitor are mixed, whereby an oil phase component mixed liquid is obtained (hereinafter, also referred to as "oil phase component mixing process"). The oil phase component mixed liquid may include other oil phase components as necessary.

Specific examples of the lycopene, (poly)glycerin fatty acid ester, other fatty acid ester component, and oxidation inhibitor that are included in the oil phase component mixed liquid used in the oil phase component mixing process, for example, the contents and preferred ranges thereof, are the same as described in connection with the various components included in the emulsion composition of the invention.

Thereafter, the oil phase component mixed liquid is heated under the temperature conditions at 90°C or higher, an oil phase composition is obtained (hereinafter, also referred to as "oil phase component heating process"). The heating temperature may be any temperature higher than or equal to 90°C, and can be set to 90°C to 155°C. From the viewpoint of suppressing thermal decomposition of lycopene, the heating temperature is preferably from 110°C to 150°C, and more preferably from 120°C to 145°C.

The heating time for the oil phase component heating process is desirably a time period in which the lycopene in the oil phase component mixed liquid is dissolved, and from the viewpoint of efficiently suppressing the non-crystallization of a crystalline body and excessive decomposition of lycopene caused by heat, the heating time is preferably from 1 minute to 60 minutes, and more preferably from 5 minutes to 45 minutes; however, the heating time is not limited to this.

An oil phase composition is obtained from an oil phase component mixed liquid containing lycopene by such a heating treatment.

In addition, regarding the heating treatment, since it is preferable to make the entire oil phase component mixed liquid to be at a uniform temperature, it is preferable to sufficiently stir the oil phase component mixed liquid while heating, and it is desirable to heat the mixed liquid with stirring in a sealed container, and to maintain the mixed liquid at a constant temperature.

An oil phase composition is obtained by the oil phase component heating process.

After the oil phase component heating process, emulsification of an oil phase composition containing the oil phase composition obtained by the oil phase component heating process and an aqueous phase composition containing a predetermined aqueous phase component (an ascorbic acid compound may also be contained) (an emulsification process) is included. By this way, an oil-in-water emulsion composition in which the oil phase component containing lycopene is finely dispersed as oil droplets (emulsion particles) in water can be obtained. In this composition, the lycopene is stably maintained.

The ratio (mass) of the oil phase and aqueous phase in the emulsification is not especially limited, and is preferably from 0.1/99.9 to 50/50, more preferably from 0.5/99.5 to 30/70, further preferably 1/99 to 20/80 as an oil phase/aqueous phase ratio (% by mass).

It is preferable to preset the oil phase/aqueous phase ratio to 0.1/99.9 or more, since the active ingredient is not lowered, and thus the practical problems of the emulsion composition tend to not occur. Furthermore, it is preferable to preset the oil phase/aqueous phase ratio to 50/50 or less, since the emulsifier concentration is not diluted, and thus the stability of the emulsion composition tends to not deteriorate.

The emulsification under pressure may be conducted by conducting an emulsification operation of one step, and it is preferable to conduct emulsification operations of two or more steps in view of obtaining homogeneous and fine emulsion particles (emulsified particles).

Specifically, it is especially preferable to use two or more kinds of emulsification apparatuses in methods including emulsifying through a high pressure homogenizer or the like, and the like, in addition to an emulsification operation of one step in which emulsification is conducted by using a general emulsification apparatus utilizing a shearing action (for example, stirring with a stirrer or an impeller, a homomixer, a continuous circulation-type shearing apparatus or the like). By using a high pressure homogenizer, the emulsion particles in the emulsion composition can further be unified into droplets of homogeneous microparticles. Furthermore, the emulsification operations can further be conducted plural times for the purpose of obtaining droplets with homogeneous particle diameters.

As the emulsification means that can be used herein, any of generally known emulsification methods such as a natural emulsification process, an interfacial chemical emulsification process, an electric emulsification process, a capillary emulsification process, a mechanical emulsification process and an ultrasonic emulsification process.

As a useful method for further miniaturization of the emulsion particles in the emulsion composition, interfacial chemical emulsification methods such as a PIT emulsification method and a gel emulsification method are known. These methods have an advantage that the consumed energy is small, and thus are suitable for the cases when a material that is susceptible to thermal deterioration is to be finely emulsified.

Alternatively, a method using a mechanical force, i. e., a method for cleaving oil droplets by applying a strong shear force from outside, is applied as a generally-used emulsification process. The most common mechanical force is a high-speed, high shear stirrer. As such stirrer, stirrers called as a Homo Mixer, a Disper Mixer and an Ultra Mixer are commercially available.

Furthermore, another mechanical emulsification apparatus useful for miniaturizaion is a high pressure homogenizer, and various apparatuses are commercially available. Since the high pressure homogenizer can apply a higher shear force as compared to a stirring system, miniaturization is possible even the amount of the emulsifier is relatively low.

High pressure homogenizers are roughly classified into a chamber type high pressure homogenizer, which has a fixed drawn part, and a homogeneous valve-type high pressure homogenizer in which the degree of opening of drawing is controlled.

Examples of the chamber type high pressure homogenizer include a MICROFLUIDIZER (manufactured by Microfluidics Corporation), a NANOMIZER (manufactured by YOSHIDA KIKAI CO., LTD.), an ULTIMIZER (manufactured by Sugino Machine, Ltd.) and the like.

Examples of the homogeneous valve-type high pressure homogenizer include a Gaulin type homogenizer (manufactured by APV), a Lanier type homogenizer (manufactured by Lanier), a high pressure homogenizer (manufactured by Niro Soavi), a homogenizer (manufactured by Sanwa Machinery Co. Ltd.), a high pressure homogenizer (manufactured by Izumi Food Machinery Co. Ltd.), an ultra high pressure homogenizer (manufactured by IKA) and the like.

As an emulsification apparatus that is a dispersion apparatus having a relatively fine energy efficiency and a simple structure, an ultrasonic homogenizer is exemplified. Examples of a high-output ultrasonic homogenizer that is also capable of production include Ultrasonic Homogenizers US-600, US-1200T, RUS-1200T and MUS-1200T (these are manufactured by Nissei Corporation), Ultrasonic Processers UIP2000, UIP-4000, UIP-8000 and UIP-16000 (these are manufactured by Hielscher) and the like. These high-output ultrasonic irradiators are used at a wavenumber of 25 kHz or less, preferably from 15 to 20 kHz.

Furthermore, as another known emulsification means, a method using a static mixer, a microchannel, a micromixer, a film emulsification apparatus or the like, which does not have any part for stirring from outside and requires only low energy, is also a useful method.

In the production method of the present embodiment, the temperature condition in the emulsification dispersion is not especially limited, and is preferably from 10°C to 100°C in view of the stability of the functionic oil-based component, and a preferable scope can be selected depending on the melting point and the like of the functionic oil-based component to be handled, if appropriate.

In the case when a high pressure homogenizer is used in the invention, the pressure is preferably 50 MPa or more, more preferably from 50 MPa to 280 MPa, and it is further preferable to conduct a treatment at from 100 MPa to 280 MPa.

Furthermore, it is preferable that the composition that has undergone the emulsification dispersion is cooled through a certain cooling apparatus within 30 seconds, preferably within 3 seconds immediately after the passage of the chamber.

In the production method of the present embodiment, in a case in which an embodiment in which the ascorbic acid compound is added to the prepared emulsion composition is adopted as an embodiment for adding the ascorbic acid compound to the aqueous phase, the ascorbic acid compound or an aqueous solution containing the ascorbic acid compound may be added to the emulsion composition obtained as mentioned above.

EXAMPLES

Hereinafter, the invention will be explained by way of Examples, but the invention is not intended to be limited to these. Note that, the "parts" and "%" in the following description are based on mass unless specified otherwise.

[Examples 1 to 8 and Comparative Examples 1 to 9]

The respective lycopene-containing emulsion compositions of Examples 1 to 8 and Comparative Examples 1 to 9 were prepared as follows.

1. Preparation of high lycopene-containing emulsions A to F

< Preparation of Emulsion A>

An oil phase composition described below was heated and mixed for 5 minutes while stirred on a hot plate at 135°C, and it was confirmed that the composition was thoroughly mixed.

An aqueous phase composition described below was heated and mixed while stirred in a constant temperature bath at 70°C, and it was confirmed that the composition was thoroughly mixed. The aqueous phase composition was maintained at 70°C.

The aqueous phase composition was added to the oil phase composition and mixed with stirring, and then the mixture was dispersed by using an ultrasonic homogenizer, whereby a crude dispersion was obtained. The obtained crude dispersion was then further subjected to high pressure emulsification by using a ultra high pressure emulsification apparatus (ULTIMIZER, manufactured by Sugino Machine, Ltd.) and setting the emulsification conditions to emulsification pressure: 100 MPa and frequency of passes (frequency of emulsification operations): 1.

As described above, Emulsion A (a lycopene-containing emulsion composition before dilution) was obtained.

- Oil phase composition -

| | |
|---|---|
| (1) Lyc-O-Mato 6% (lycopene content: 6%)[*1] | 5.7 parts |
| (2) Glycerin tri(caprylate/caprate) [*2] | 8.4 parts |
| (3) Mixed tocopherol[*3] | 0.64 parts |
| (4) Diglyceryl monostearate[*4] | 0.28 parts |

*1: "LYC-O-MATO 6%" manufactured by Sunbright Co., Ltd., an extractive matter derived from tomato
*2: "COCONARD MT" (HLB = 1) manufactured by Kao Corporation
*3: "RIKEN E OIL 800" manufactured by Riken Vitamin Co., Ltd.
*4: "NIKKOL DGMS" (HLB = 5.0) manufactured by Nikko Chemicals Co., Ltd.

**[0203]** - Aqueous phase composition -

| | |
|---|---|
| (1) Decaglyceryl oleate[*5] | 10 parts |
| (2) Glycerin | 45.0 parts |
| (3) Purified water | 30.0 parts |

*5: "DECAGLYN 1-O" manufactured by Nikko Chemicals Co., Ltd.

<Preparation of Emulsions B to D>

Emulsions B to D were respectively obtained in a same manner as in Emulsion A, except that the emulsification conditions (emulsification pressure and frequency of passes) applied to the high pressure emulsification in the preparation of Emulsion A were changed as shown in Table 1.

<Preparation of Emulsion E>

Emulsion E was obtained in a similar manner as in Emulsion A, except that the hot plate temperature in the preparation of the oil phase composition in the preparation of Emulsion A was changed to 70°C, and the emulsification conditions (emulsification pressure and frequency of passes) applied to the high pressure emulsification in the preparation of Emulsion A were changed as shown in Table 1.

<Preparation of Emulsion F>

Emulsion F was obtained in the same manner as in Emulsion A, except that the kinds and contents of the respective components used in the preparation of the oil phase composition, and the emulsification conditions (emulsification pressure and frequency of passes) applied to the high pressure emulsification, in the preparation of Emulsion A, were changed as shown in Table 1.

As the ascorbic acid shown in Table 1, "L(+)-ascorbic acid", which is an agent manufactured by Wako Pure Chemical Industries, Ltd., was used.

The numerical values that show the contents of the respective components in Table 1 represent "parts".

[Table 1]

| | | | High lycopene-containing emulsion | | | | | (Parts) |
|---|---|---|---|---|---|---|---|---|
| | | | A | B | C | D | E | F |
| Oil phase | LCY O-M ATO 6% | | 5.70 | 5.70 | 5.70 | 5.70 | 5.70 | 5.70 |
| | COCONARD MT | | 8.4 | 8.4 | 8.4 | 8.4 | 8.4 | 7.7 |
| | Mix tocopherol | | 0.64 | 0.64 | 0.64 | 0.64 | 0.64 | 0.64 |
| | Diglycerin monostearate | | 0.28 | 0.28 | 0.28 | 0.28 | 0.28 | 0.28 |
| | Ascorbic acid | | | | | | | 0.7 |
| Aqueous phase | Decaglyceryl oleate | | 10.0 | 10.0 | 10.0 | 10.0 | 10.0 | 10.0 |
| | Glycerin | | 45.0 | 45.0 | 45.0 | 45.0 | 45.0 | 45.0 |
| | Water | | 30.0 | 30.0 | 30.0 | 30.0 | 30.0 | 30.0 |
| Emulsification pressure | | | 100MPA | 180MPa | 245MPa | 245MPa | 245MPa | 245MPa |
| Frequency of passes | | | 1 | 1 | 1 | 3 | 3 | 3 |
| Particle diameter immediately after preparation | | | 160 nm | 115 nm | 98 nm | 67 nm | 115 nm | 112nm |
| Presence or absence of crystal (observed under polarized light microscope) | | | Absent | Absent | Absent | Absent | Present | Absent |

For the high lycopene-containing emulsions A to F obtained above, the particle diameter immediately after the preparation, and the presence or absence of a crystal were confirmed as follows. The results are described together in Table 1.

- Particle diameter -

Each of Emulsions A to F was filled in a 5 ml glass vial to prepare a sample. Purified water was added to each sample to prepare a 0.33% dilution liquid, and the average particle diameter (median particle diameter) of the particles on the volume basis in the dilution liquid was measured by a dynamic light scattering meter (trade name: FPAR-1000, manufactured by Otsuka Electronics Co., Ltd.).

- Presence or absence of crystal -

Using a polarized light microscope (product name: PCLIPSE LV100POL, manufactured by Nikon Corporation), each of the emulsions A to J was visually observed, and the presence or absence of a crystal derived from the lycopene existing in the emulsion was confirmed according to the following criteria. The observation criteria are as follows. Absent: a crystalline derived from lycopene is not observed.
Present: a crystalline derived from lycopene is observed.

2. Preparation of lycopene-containing emulsion composition

Using the high lycopene-containing emulsions A to F obtained above, and the diluted liquids having the compositions shown in the following Table 2 or 3 (aqueous solutions each containing an ascorbic acid compound), the respective lycopene-containing emulsion compositions of Examples 1 to 8 and Comparative Examples 1 to 9 were prepared by mixing under stirring at 25°C so as to give the compositions described in the following Table 2 or 3.
The following compounds were used for the various components described in Tables 2 and 3.

- Ascorbic acid: "L(+)-ascorbic acid" manufactured by Wako Pure Chemical Industries, Ltd.
- Mg ascorbyl phosphate: "magnesium L-ascorbyl phosphate" manufactured by Wako Pure Chemical Industries, Ltd.
- Na ascorbate: "sodium L(+)-ascorbate" manufactured by Wako Pure Chemical Industries, Ltd.

- Citric acid: "citric acid" manufactured by Wako Pure Chemical Industries, Ltd.

The numerical values that indicate the incorporation amounts of the respective components in Table 2 or 3 indicate "parts".

3. Evaluation

For the respective lycopene-containing emulsion compositions of the Examples and Comparative Examples obtained as described above, as the indices for storage stability, (1) measurement of the emulation particle diameter and (2) evaluation of the lycopene decomposition ratio, were carried out. The evaluation results are described together in Tables 2 and 3.

(1) Measurement of emulsion particle diameter

Each of the lycopene-containing emulsion compositions of the Examples and Comparative Examples obtained as described above was divided into two portions, the portions were respectively filled into 5 ml glass vials, and one of the vials was stored at 50°C for 2 weeks in a dark place. For each of the emulsion compositions immediately after preparation and after storage for 2 weeks, the average particle diameter (median particle diameter) based on the volume of the particles in the sample was measured by a dynamic light scattering meter (trade name: FPAR-1000, manufactured by Otsuka Electronics, Co., Ltd.)

(2) Lycopene decomposition ratio

The lycopene decomposition ratio was measured by using absorbance, and the stability over time of the lycopene was evaluated.
Each of the lycopene-containing emulsion compositions of the Examples and Comparative Examples obtained above was divided into two portions, the portions were respectively filled into 5 ml glass vials, and one of the vials was stored at 50°C for 2 weeks in a dark place, whereby samples for evaluation were prepared.
For each of the samples immediately after preparation and after storage for 2 weeks, absorbance was measured according to the following measurement conditions, and a lycopene decomposition ratio (%) was calculated by the following formula.

$$\text{Decomposition ratio (\%)} = (\text{absorbance immediately after preparation - absorbance after storage } 50°C \text{ for 2 weeks}) / \text{absorbance immediately after preparation} \times 100$$

A lower decomposition ratio (%) indicates superior stability over time.

(Conditions for measurement of absorbance)

The emulsion composition was diluted with pure water so that the lycopene concentration in the composition became 0.0004%. For each diluted emulsion composition, absorbance at 510 nm was measured in a 10 mm cell using a UV-VIBLE spectrum photometer UV-2550 (manufactured by Shimadzu Corporation). In a case in which the lycopene concentration in the emulsion composition was 0.0004% or less, the neat liquid was directly measured.

[Table 2]

| | | Example 1 | Example 2 | Example 3 | Example 4 | Example 5 | Example 6 | Example 7 | Example 8 |
|---|---|---|---|---|---|---|---|---|---|
| High lycopene-containing emulsion | Emulsion A | | | | | | | | |
| | Emulsion B | | | | 14 | | | | |
| | Emulsion C | | | | | 14 | | | |
| | Emulsion D | 28 | 14 | 0.1 | | | 14 | 14 | |
| | Emulsion E | | | | | | | | 14 |
| | Emulsion F | | | | | | | | |
| Diluted liquid | Ascorbic acid | | | | | | 0.1 | | |
| | Mg ascorbyl phosphate | 1.0 | 1.0 | 1.0 | 1.0 | 1.0 | | | 1.0 |
| | Na ascorbate | | | | | | | 0.1 | |
| | Na citrate | 1.0 | 1.0 | 1.0 | 1.0 | 1.0 | 1.0 | 1.0 | 1.0 |
| | pH 7.0 0.10 mM phosphate buffer aqueous solution | 70.0 | 84.0 | 97.9 | 84.0 | 84.0 | 84.9 | 84.9 | 84.0 |
| Lycopene content | | 0.096% | 0.048% | 0.0003% | 0.048% | 0.048% | 0.048% | 0.048% | 0.048% |
| Particle diameter immediately after preparation | | 67 nm | 67 nm | 66 nm | 116 nm | 98 nm | 67 nm | 68 nm | 115 nm |
| Particle diameter after storage at 50°C for 2 weeks | | 80 nm | 68 nm | 68 nm | 117 nm | 98 nm | 68 nm | 68 nm | 118nm |
| Lycopene decomposition ratio after storage at 50°C for 2 weeks | | 12% | 8% | 9% | 12% | 10% | 7% | 8% | 15% |

[Table 3]

| | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 |
|---|---|---|---|---|---|---|---|---|---|---|
| High lycopene-containing emulsion | Emulsion A | 35 | 28 | 14 | 0.2 | 14 | | | | |
| | Emulsion B | | | | | | | | | |
| | Emulsion C | | | | | | | | | |
| | Emulsion D | | | | | | 14 | | 35 | 35 |
| | Emulsion E | | | | | | | | | |
| | Emulsion F | | | | | | | 14 | | |
| Diluted liquid | Ascorbic acid | | | | | | | | | |
| | Mg ascorbyl phosphate | 1.0 | 1.0 | 1.0 | 1.0 | | | | 1.0 | |
| | Na ascorbate | | | | | | | | | |
| | Na citrate | 1.0 | 1.0 | 1.0 | 1.0 | 1.0 | 1.0 | 1.0 | 1.0 | 1.0 |
| | pH 7.0 0.10 mM phosphate buffer aqueous solution | 63.0 | 70.0 | 84.0 | 97.8 | 85.0 | 85.0 | 85.0 | 63.0 | 64.0 |
| Lycopene content | | 0.120% | 0.096% | 0.048% | 0.001% | 0.048% | 0.048% | 0.048% | 0.120% | 0.120% |
| Particle diameter immediately after preparation | | 165nm | 162 nm | 160 nm | 160 nm | 160 nm | 68 nm | 118nm | 70 nm | 68 nm |
| Particle diameter after storage at 50°C for 2 weeks | | 250 nm | 180 nm | 165 nm | 161 nm | 162 nm | 69 nm | 119 nm | 140 nm | 69 nm |
| Lycopene decomposition ratio after storage at 50°C for 2 weeks | | 29% | 27% | 26% | 26% | 29% | 38% | 37% | 21% | 34% |

EP 2 902 101 A1

As shown in Tables 2 and 3, it is understood that either of the respective lycopene-containing emulsion compositions of the Examples is excellent in storage stability, since an emulsification state containing fine emulsion particles of 120 nm or less is maintained even after storage, and the decomposition ratio of the lycopene is low.

In more detail, the following matters are found.

It is understood by comparing the differences between Examples 1 to 3 and Comparative Example 8, and the differences between Comparative Example 3 and Comparative Example 1, that the storage stability is significantly improved by adjusting the particle diameter of the emulsion particles to within the scope of the invention, and by adjusting the content of the lycopene to within the scope of the invention.

It is understood by comparing the differences between Example 2 and Comparative Example 6, and the differences between Comparative Example 3 and Comparative Example 5, that the storage stability is significantly improved by adjusting the particle diameter of the emulsion particles to within the scope of the invention, and by adding the ascorbic acid compound to the aqueous phase.

It is understood by comparing the differences between Example 2 and Comparative Example 3, the differences between Comparative Example 6 and Comparative Example 5, and the differences between Comparative Example 8 and Comparative Example 1, that the storage stability is significantly improved by adjusting the particle diameter of the emulsion particles to within the scope of the invention, only after incorporating the ascorbic acid compound in the aqueous phase and adjusting the content of the lycopene towithin the scope of the invention.

It is understood by comparing Example 8 and Comparative Example 7 that the stability is significantly improved only after adding the ascorbic acid compound to the aqueous phase.

It is understood by comparing Example 1 (lycopene content: 0.096%) and Example 2 (lycopene content: 0.048%) that a lycopene content of 0.05% or less is more preferable than 0.1% or less.

It is understood by the comparison of Example 4 (Emulsion B was used) and Example 8 (Emulsion E was used) that it is more preferable for the lycopene to be in a non-crystalline state.

The disclosure of Japanese Patent Application No. 2012-217835 is herein incorporated by reference. All of the documents, patent applications and technical specifications described in this specification are herein incorporated by reference as if each of the individual documents, patent applications and technical specifications were specifically and individually indicated to be incorporated by reference herein.

**Claims**

1. A lycopene-containing oil-in-water emulsion composition, comprising:

   an oil phase containing lycopene in an amount of from 0.00001 % by mass to 0.1 % by mass with respect to a total mass of the emulsion composition; and
   an aqueous phase containing at least one selected from the group consisting of ascorbic acid, derivatives thereof, salts of ascorbic acid and salts of derivatives of ascorbic acid,
   wherein an average particle diameter of emulsion particles is 120 nm or less.

2. The lycopene-containing oil-in-water emulsion composition according to claim 1, comprising the lycopene in a non-crystalline state.

3. The lycopene-containing oil-in-water emulsion composition according to claim 1 or 2, further comprising an emulsifier having an HLB of 10 or more in an amount of from 50% by mass to 2,000% by mass with respect to an amount of the oil phase and from 0.1% by mass to 15% by mass with respect to the total mass of the emulsion composition.

4. The lycopene-containing oil-in-water emulsion composition according to claim 3, wherein the emulsifier having an HLB of 10 or more is a polyglycerin fatty acid ester.

5. The lycopene-containing oil-in-water emulsion composition according to any one of claims 1 to 4, further comprising a fatty acid ester component having a total carbon number of 10 to 60 and having no hydroxyl group in the molecule.

6. The lycopene-containing oil-in-water emulsion composition according to any one of claims 1 to 5, wherein the emulsion particles have an average particle diameter of from 5 nm to 100 nm.

7. The lycopene-containing oil-in-water emulsion composition according to any one of claims 1 to 6, wherein the content of the lycopene is from 0.0002% by massto 0.05% by mass with respect to the total mass of the emulsion composition.

8. The lycopene-containing oil-in-water emulsion composition according to any one of claims 1 to 7, wherein the at least one selected from the group consisting of ascorbic acid, derivatives thereof, salts of ascorbic acid and salts of derivatives of ascorbic acid, is ascorbic acid, sodium ascorbate or a combination thereof.

9. A method of producing the lycopene-containing oil-in-water emulsion composition according to any one of claims 1 to 8, the method comprising:

preparing an oil-in-water emulsion composition containing lycopene; and
adding at least one selected from the group consisting of ascorbic acid, derivatives thereof, salts of ascorbic acid and salts of derivatives of ascorbic acid, to the oil-in-water emulsion composition.

## INTERNATIONAL SEARCH REPORT

| | International application No. |
|---|---|
| | PCT/JP2013/076419 |

A.   CLASSIFICATION OF SUBJECT MATTER
*B01J13/00*(2006.01)i, *A23D7/00*(2006.01)i, *A23L1/30*(2006.01)i, *A61K8/06*
(2006.01)i, *A61K8/31*(2006.01)i, *A61K8/67*(2006.01)i

According to International Patent Classification (IPC) or to both national classification and IPC

B.   FIELDS SEARCHED

Minimum documentation searched (classification system followed by classification symbols)
B01J13/00, A23D7/00, A23L1/30, A61K8/06, A61K8/31, A61K8/67

Documentation searched other than minimum documentation to the extent that such documents are included in the fields searched
Jitsuyo Shinan Koho          1922-1996   Jitsuyo Shinan Toroku Koho   1996-2013
Kokai Jitsuyo Shinan Koho    1971-2013   Toroku Jitsuyo Shinan Koho   1994-2013

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| X | JP 2008-169117 A  (Fujifilm Corp.),<br>24 July 2008 (24.07.2008),<br>claims 1, 4, 8; paragraphs [0009], [0029] to<br>[0032], [0035], [0038], [0044], [0084]<br>& US 2010/0021511 A1     & EP 2116223 A1<br>& WO 2008/081905 A1      & CN 101573100 A<br>& KR 10-2009-0107036 A | 1-9 |
| A | JP 2011-241177 A  (Fujifilm Corp.),<br>01 December 2011 (01.12.2011),<br>entire text<br>& US 2013/0071451 A1     & EP 2574337 A1<br>& WO 2011/145659 A1      & CN 102892414 A<br>& KR 10-2013-0079401 A | 1-9 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an i nventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 December, 2013 (12.12.13) | 24 December, 2013 (24.12.13) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2013/076419

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2012-184221 A  (Fujifilm Corp.),<br>27 September 2012 (27.09.2012),<br>claims 1 to 13<br>(Family: none) | 1-9 |

Form PCT/ISA/210 (continuation of second sheet) (July 2009)

**REFERENCES CITED IN THE DESCRIPTION**

**Patent documents cited in the description**

- JP 2011241177 A
- JP 2002078447 A

- JP 2012217835 A